# EUROPEAN PATENT APPLICATION

(11) **EP 2 642 546 A1**
(43) Date of publication of application: **25.09.2013**
(21) Application number: 11841214.7
(22) Date of filing: 15.11.2011
(51) Int. Cl.: H01L 51/42, B01F 17/32, C07D 417/14, C09D 11/00

(54) **INK FOR ACTIVE LAYER OF ORGANIC SOLAR CELL, ORGANIC SOLAR CELL, AND PROCESS FOR MANUFACTURE OF ORGANIC SOLAR CELL**

(30) Priority: 16.11.2010 JP 2010256179; 16.11.2010 JP 2010256180; 23.02.2011 JP 2011037475; 28.03.2011 JP 2011070953
(71) Applicant: Sekisui Chemical Co., Ltd., Osaka-shi, Osaka 530-0047 (JP)
(72) Inventor: HAYAKAWA Akinobu, Mishima-gun Osaka 618-0021 (JP); ITO Kazushi, Mishima-gun Osaka 618-0021 (JP); SASAKI Taku, Mishima-gun Osaka 618-0021 (JP)
(74) Representative: Hart-Davis, Jason
(86) International application number: PCT/JP2011/076331
(87) International publication number: WO 2012/067124

(57) **Abstract**

The present invention aims to provide an ink for an active layer of an organic solar cell, wherein an active layer having high energy conversion efficiency can be stably and easily formed from the ink; an organic solar cell having high energy conversion efficiency; and a method for producing the organic solar cell. A first aspect of the present invention is an ink for an active layer of an organic solar cell, the ink comprising: an organic semiconductor compound; an inorganic semiconductor compound; an organic solvent; and a dispersant; wherein the dispersant is a compound having a structure with an aromatic ring and/or heterocyclic ring and a polar group asymmetrically bonded to the structure, and fulfills all of the following requirements (1) to (3):
(1) the dispersant has a lower LUMO level than the organic semiconductor compound;
(2) solubility of the dispersant in the organic solvent is equal to or higher than solubility of the organic semiconductor compound in the organic solvent; and
(3) the dispersant has a higher HOMO level than the inorganic semiconductor compound.

## Description

### TECHNICAL FIELD

The present invention relates to an ink for an active layer of an organic solar cell, wherein an active layer having high energy conversion efficiency can be stably and easily formed with the ink; an organic solar cell having high energy conversion efficiency; and a method for producing the organic solar cell.

### BACKGROUND ART

Heretofore, organic solar cells including a laminate of an organic semiconductor layer and an inorganic semiconductor layer and electrodes at both sides of the laminate have been developed. In such organic solar cells having the above structure, photocarriers (electron-hole pairs) are generated by photoexcitation in the organic semiconductor layer, and an electric field is generated by the transfer of electrons through the inorganic semiconductor layer and the transfer of holes through the organic semiconductor layer. However, in the organic semiconductor layer, an active region for photocarrier generation is very narrow, i.e., about several tens of nanometers around the junction interface between the organic semiconductor layer and the inorganic semiconductor layer, and the rest of the organic semiconductor layer cannot contribute to photocarrier generation. Accordingly, these solar cells unfortunately have low energy conversion efficiency.

In order to solve the above problem, the use of a composite film in which an organic semiconductor and an inorganic semiconductor are mixed and combined has been examined.
For example, Patent Literature 1 discloses an organic solar cell including: a co-evaporated thin film in which an organic semiconductor and an inorganic semiconductor are formed as a composite by co-evaporation; and electrodes made of semiconductor, metal, or a combination thereof arranged so as to sandwich the thin film therebetween in order to provide a built-in electric field to the composite thin film. According to Patent Literature 1, because the organic/inorganic composite thin film disclosed therein is configured such that a p-n junction (organic/inorganic semiconductor junction) is formed entirely in the film, the entire film plays an active role in photocarrier generation and the light absorbed by the film entirely contributes to carrier generation, thus creating an effect of producing a large photocurrent.

Another attempt has also been made to improve energy conversion efficiency by tightly packing an organic semiconductor in an inorganic semiconductor.
For example, Patent Literature 2 discloses an organic solar cell including an active layer between two electrodes, the active layer containing an organic electron donor and a compound semiconductor crystal, wherein the organic electron donor and the compound semiconductor crystal are mixed and dispersed in the active layer, and the compound semiconductor crystal contains two types of rod-shaped crystals having different average particle sizes, with the average particle sizes and the content ratio of these two types of rod-shaped crystals being in specific ranges. Patent Literature 2 states that the packing ratio of the compound semiconductor crystal in the active layer can be increased, and that a solar cell having high conversion efficiency can thereby be obtained.

However, even the organic solar cell disclosed in Patent Literature 1 or 2 still has considerably low energy conversion efficiency. Thus, further improvement in the energy conversion efficiency is necessary in order to develop organic solar cells that can be applicable in practical use.

### CITATION LIST

### - Patent Literature

Patent Literature 1: Japanese Kokai Publication No. 2002-100793
Patent Literature 2: Japanese Patent No. 4120362

### SUMMARY OF THE INVENTION

### - Technical Problem

The present invention aims to provide an ink for an active layer of an organic solar cell, wherein an active layer having high energy conversion efficiency can be stably and easily formed with the ink; an organic solar cell having high energy conversion efficiency; and a method for producing the organic solar cell.

### - Solution to Problem

A first aspect of the present invention is an ink for an active layer of an organic solar cell, the ink containing an organic semiconductor compound, an inorganic semiconductor compound, an organic solvent, and a dispersant; wherein the dispersant is a compound having a structure with an aromatic ring and/or heterocyclic ring and a polar group asymmetrically bonded to the structure, and fulfills all of the following requirements (1) to (3):
(1) the dispersant has a lower LUMO level than the organic semiconductor compound;
(2) solubility of the dispersant in the organic solvent is equal to or higher than solubility of the organic semiconductor compound in the organic solvent; and
(3) the dispersant has a higher HOMO level than the inorganic semiconductor compound.

A second aspect of the present invention is an organic solar cell having an active layer in which an inorganic semiconductor compound is present in an organic semiconductor compound, wherein in a cross-section of the active layer in a thickness direction, the inorganic semiconductor compound has an area ratio of 75 to 100% in a region from a cathode-side surface to a depth of 20% of a film thickness.
The present invention is described in detail below.

The present inventors found that it is possible to obtain an ink for an active layer of an organic solar cell, wherein an active layer having high energy conversion efficiency can be stably and easily formed from the ink, by adding a dispersant that fulfills specific requirements to an ink for an active layer of an organic solar cell, the ink containing an organic semiconductor compound, an inorganic semiconductor compound, and an organic solvent. The first aspect of the present invention is thus accomplished.
Further, the present inventors found that electron paths are easily formed by setting the area ratio of the inorganic semiconductor compound in a specific range in a region from a cathode-side surface to a depth of 20% of a film thickness, in a cross section of the active layer in a thickness direction, the active layer being formed such that the inorganic semiconductor compound is present in the organic semiconductor compound; and that this results in an increase in the photocurrent value and significant improvement in energy conversion efficiency. The second aspect of the present invention is thus accomplished.

First, an ink for an active layer of an organic solar cell of the first aspect is described.
The ink for an active layer of an organic solar cell of the first aspect contains an organic semiconductor compound.
The organic semiconductor compound is not particularly limited. Examples include conductive polymers such as poly(3-alkylthiophene), polyparaphenylene vinylene derivatives, polyvinylcarbazole derivatives, polyaniline derivatives, and polyacetylene derivatives; phthalocyanine derivatives; naphthalocyanine derivatives; pentacene derivatives; and porphyrin derivatives. Preferred among the above are conductive polymers because activity layers with high hole mobility can be formed therefrom. Poly(3-alkylthiophene) is more preferred.

The ink for an active layer of an organic solar cell of the first aspect contains an inorganic semiconductor compound.
The inorganic semiconductor compound is not particularly limited. Examples include titanium oxide, zinc oxide, tin oxide, indium oxide, gallium oxide, antimony oxide, tungsten oxide, silicon oxide, aluminum oxide, barium titanate, strontium titanate, cadmium sulfide, zinc sulfide, tin sulfide, antimony sulfide, bismuth sulfide, indium sulfide, silicon sulfide, and vanadium oxide. Examples of the inorganic semiconductor compounds include compounds containing elements of group 13 and elements of group 15 of the periodic table, such as InP, InAs, GaP, and GaAs, and compounds containing elements of group 12 and elements of group 16 of the periodic table, such as CdSe, CdTe, and ZnS. These inorganic semiconductor compounds may be those in which two or more of the above components are mixed, or compounds doped with an element different from a main component. These inorganic semiconductor compounds may be used alone or in combination of two or more thereof. Preferred among the above are zinc oxide, tin oxide, indium oxide, antimony oxide, zinc sulfide, tin sulfide, antimony sulfide, and bismuth sulfide because active layers having high electron mobility can be formed from these compounds.

The shape of the inorganic semiconductor compound is not particularly limited. Examples include a rod shape and a spherical shape, with a spherical shape being preferred.
In the case where the inorganic semiconductor compound has a spherical shape, the average particle size is preferably 1 to 50 nm, and the ratio of average particle size/average crystallite size is preferably 1 to 3. Because the inorganic semiconductor compound has such an average particle size and such a ratio of average particle size/average crystallite size, when electrons pass through the inorganic semiconductor compound in the active layer formed from the ink for an active layer of an organic solar cell, the transfer of electrons is less likely to be impeded by grain boundaries, and electrons are smoothly collected at the electrode. Consequently, recombination of electrons and holes is inhibited, resulting in a further improvement in energy conversion efficiency.

The average particle size of less than 1 nm leads to an increased number of grain boundaries between particles of the inorganic semiconductor compound in the active layer formed from the ink for an active layer of an organic solar cell, possibly resulting in more impediments to the transfer of electrons. With the average particle size of more than 50 nm, photocarriers generated in the organic semiconductor compound may not be efficiently transferred to the junction interface between the organic semiconductor compound and the inorganic semiconductor compound in the active layer formed from the ink for an active layer of an organic solar cell. The lower limit of the average particle size of the inorganic semiconductor compound is more preferably 2 nm, still more preferably 3 nm. The upper limit thereof is more preferably 30 nm, still more preferably 25 nm, and particularly preferably 20 nm. The "average particle size" as used herein can be measured, for example, using a dynamic light scattering analyzer (380DLS produced by PSS NICOMP).

With the ratio of average particle size/average crystallite size of more than 3, the transfer of electrons is impeded by grain boundaries in the particles, and electrons and holes may be easily recombined. The more preferred upper limit of the ratio of average particle size/average crystallite size of the inorganic semiconductor compound is 2.5.

The preferred lower limit of the average crystallite size of the inorganic semiconductor compound is 1 nm. With the average crystallite size of less than 1 nm, the transfer of electrons may be impeded by grain boundaries in the particles, and electrons and holes may be easily recombined.
The term "crystallite size" as used herein refers to the size of a crystallite calculated using Scherrer's X-ray diffraction method. Additionally, the average crystallite size can be measured using, for example, an X-ray diffractometer (RINT1000 produced by Rigaku Corporation).

Examples of the methods for preparing particles of the inorganic semiconductor compound include, in the case of producing inorganic semiconductor compound particles of zinc oxide, a method for preparing a dispersion of inorganic semiconductor compound particles by adding a zinc metal salt to an organic solvent, adding an alkaline compound to the mixture while stirring the same in a hot-water bath, and stirring the resulting mixture. In the case of using the above-described method, the range of the ratio of average particle size/average crystallite size can be adjusted by changing the temperature of the hot-water bath.
The following methods are also applicable as the methods for preparing particles of the inorganic semiconductor compound: dry methods such as flame spray pyrolysis, CVD, PVD, and grinding; and wet methods such as reduction, microemulsion, hydrothermal reaction, and sol-gel.

The ratio of the amount of the inorganic semiconductor compound to the amount of the organic semiconductor compound is not particularly limited. The preferred lower limit of the amount of the inorganic semiconductor compound is 50 parts by weight, and the preferred upper limit of the amount of the inorganic semiconductor compound is 1,000 parts by weight, respectively, based on 100 parts by weight of the organic semiconductor compound. In the case where the amount of the inorganic semiconductor compound is less than 50 parts by weight, it may result in insufficient electron transfer in the active layer formed from the ink for an active layer of an organic solar cell. In the case where the amount of the inorganic semiconductor compound is more than 1,000 parts by weight, it may result in insufficient hole transfer in the active layer formed from the ink for an active layer of an organic solar cell. The more preferred lower limit of the amount of the inorganic semiconductor compound is 100 parts by weight, and the more preferred upper limit of the amount of the inorganic semiconductor compound is 500 parts by weight, based on 100 parts by weight of the organic semiconductor compound.

The ink for an active layer of an organic solar cell of the first aspect contains an organic solvent.
The organic solvent is not particularly limited. Chlorobenzene, chloroform, methyl ethyl ketone, toluene, ethyl acetate, ethanol, xylene, and the like are preferred.

The amount of the organic solvent is not particularly limited. The preferred lower limit is 20 parts by weight, and the preferred upper limit is 1,000 parts by weight, respectively, per part by weight of the organic semiconductor compound. In the case where the amount of the organic solvent is less than 20 parts by weight, the viscosity of the ink for an active layer of an organic solar cell may be too high, and it may be impossible to stably and easily form an active layer. In the case where the amount of the organic solvent is more than 1,000 parts by weight, the viscosity of the ink for an active layer of an organic solar cell may be too low, and it may be impossible to form an active layer having a sufficient thickness. The more preferred lower limit of the amount of the organic solvent is 50 parts by weight, and the more preferred upper limit of the amount of the organic solvent is 500 parts by weight, per part by weight of the organic semiconductor compound.

The ink for an active layer of an organic solar cell of the first aspect of the present invention contains a dispersant.
The dispersant is a compound having a structure with an aromatic ring and/or heterocyclic ring and a polar group asymmetrically bonded to the structure. Examples of the polar group include hydrophilic groups such as carboxyl, amino, cyano, isocyanate, and isothiocyanate groups. Carboxyl groups are preferred.
The expression "a polar group asymmetrically bonded to the structure" as used herein means that the dispersant has only one polar group in the molecule, or has two or more polar groups in the molecule and these two or more polar groups are not symmetrically positioned in the structural formula. The term "symmetrically positioned" herein means that the centers of two or more polar groups coincide with the center of the molecule.

Because the dispersant is a compound having a structure with an aromatic ring and/or heterocyclic ring and a polar group asymmetrically bonded to the structure, it can function as a dispersant for increasing the dispersibility of the organic semiconductor compound and the inorganic semiconductor compound in the ink for an active layer of an organic solar cell of the first aspect of the present invention. Therefore, the organic semiconductor compound and the inorganic semiconductor compound are extremely well dispersed in the active layer formed from the ink for an active layer of an organic solar cell of the first aspect of the present invention. Further, in the active layer, the area of the junction interface between the organic semiconductor compound and the inorganic semiconductor compound is large, and the active region for photocarrier generation is large. Accordingly, an active layer having high energy conversion efficiency can be formed by the use of the ink for an active layer of an organic solar cell of the first aspect of the present invention.
In the case where the dispersant does not have a structure with an aromatic ring and/or heterocyclic ring or does not have a polar group; or in the case where polar groups are symmetrically bonded to the structure with an aromatic ring and/or heterocyclic ring, the organic semiconductor compound and the inorganic semiconductor compound in the ink for an active layer of an organic solar cell have low dispersibility.

The use of the ink for an active layer of an organic solar cell of the first aspect of the present invention also allows the active layer to be formed by printing techniques such as spin coating. Because the organic semiconductor compound and the inorganic semiconductor compound have high dispersibility and a printing technique can be employed as the method for forming the active layer, the active layer can be stably and easily formed using the ink for an active layer of an organic solar cell of the first aspect of the present invention, and the cost of forming the active layer can be reduced.

The dispersant is preferably a compound having a nitrogen atom, a sulfur atom, a fluorine atom, or a carbonyl group at a position other than the position of the polar group bonded to the structure. Because the dispersant is such a compound, electrons can easily move to the inorganic semiconductor compound in the active layer formed from the ink for an active layer of an organic solar cell, thus further improving energy conversion efficiency. In particular, the dispersant is more preferably a compound having a carbonyl group at a position other than the position of the polar group bonded to the structure.

The dispersant is preferably a compound having an electron donor site and an electron acceptor site. Because the dispersant is a compound having an electron donor site and an electron acceptor site, electrons can easily move from the organic semiconductor compound to the inorganic semiconductor compound in the active layer formed from the ink for an active layer of an organic solar cell, thus further improving energy conversion efficiency.
The terms "electron donor site" and "electron acceptor site" as used herein respectively refer to a site having electron donating properties and a site having electron accepting properties (electron withdrawing properties). Specifically, the electron donor site has relatively higher HOMO and LUMO levels compared to the electron acceptor site. In contrast, the electron acceptor site has relatively lower HOMO and LUMO levels compared to the electron donor site.

The electron donor site and the electron acceptor site are preferably conjugated with each other, i.e., connected through a conjugated bond. The electron donor site and the electron acceptor site may be adjacent to each other, or an optionally branched alkyl group, arylene group, or the like having 2 or more carbon atoms may be interposed between the two sites.

Specific examples of the electron donor site include structures represented by the following formulae (a-1) to (a-16).

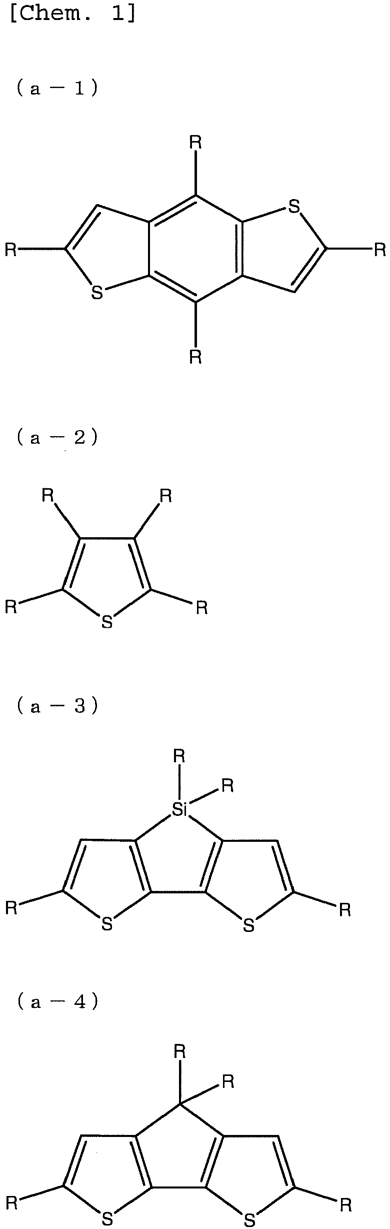

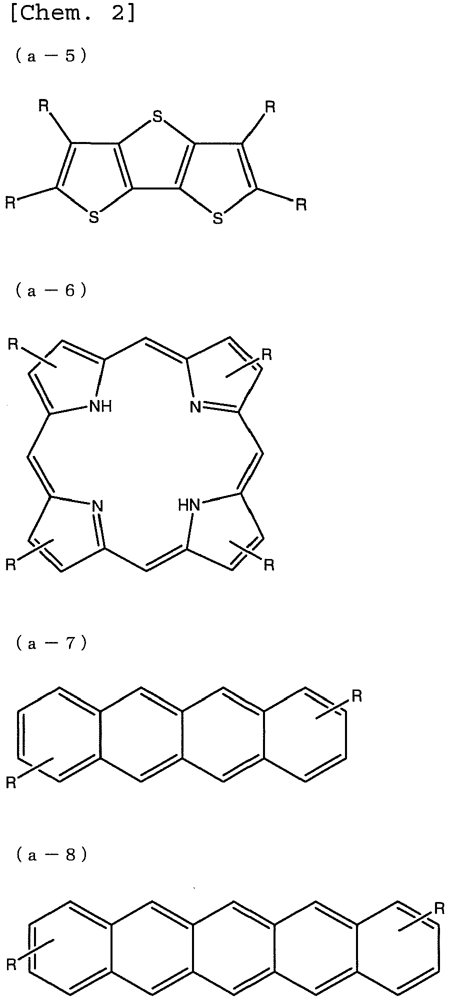

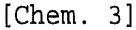

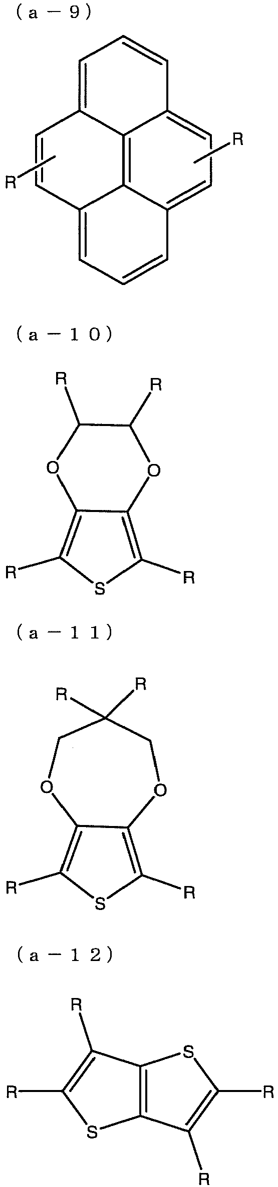

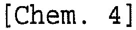

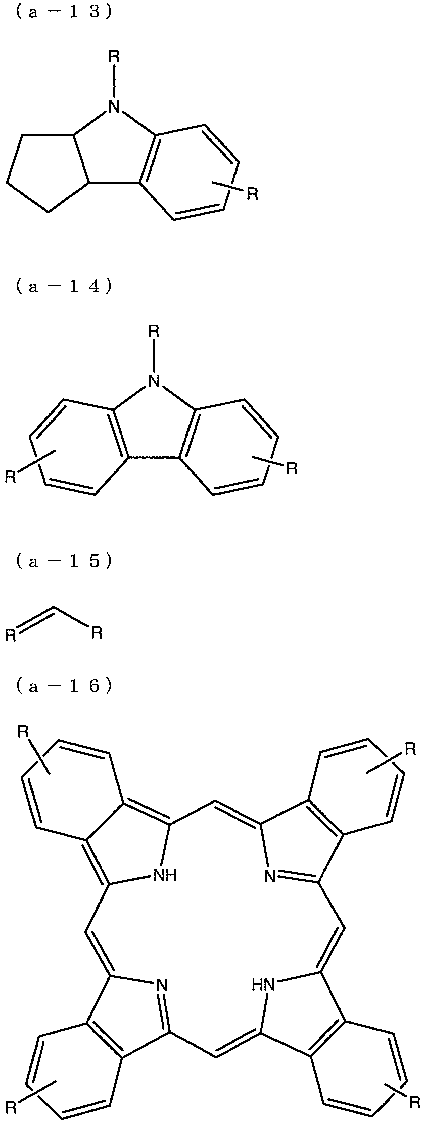

In formulae (a-1) to (a-16), R represents a hydrogen atom or a functional group. Examples of the functional group represented by R in formulae (a-1) to (a-16) include alkyl, aryl, alkoxy, alkenyl, alkynyl, and heteroaryl groups. Further, the functional group represented by R in formulae (a-1) to (a-16) may be an electron donor site or an electron acceptor site.

Specific examples of the electron acceptor site include structures represented by the following formulae (b-1) to (b-14).

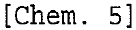

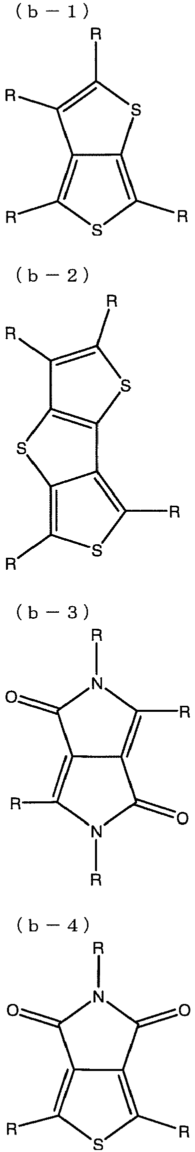

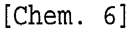

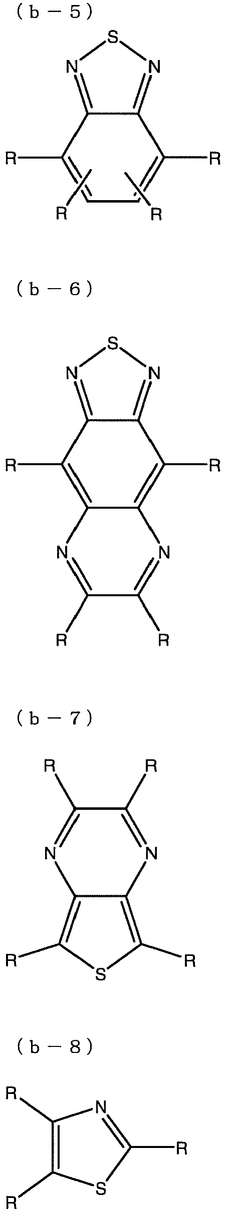

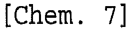

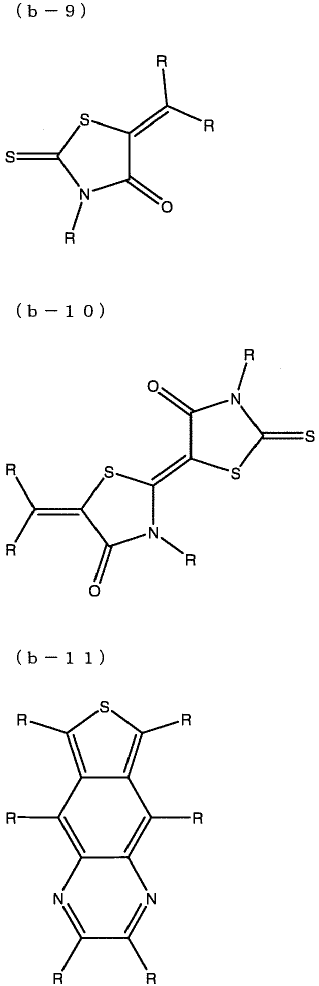

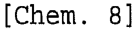

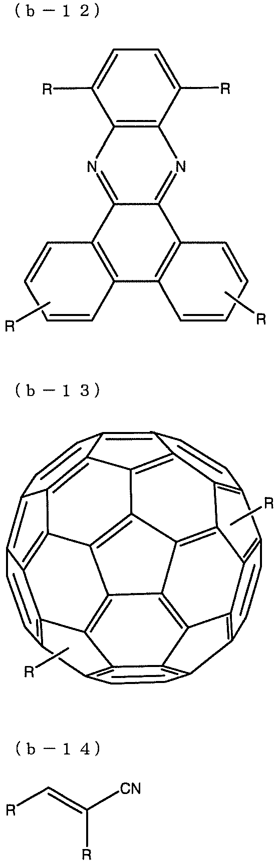

In formulae (b-1) to (b-14), R represents a hydrogen atom or a functional group. Examples of the functional group represented by R in formulae (b-1) to (b-14) include alkyl, aryl, alkoxy, alkenyl, alkynyl, and heteroaryl groups. The functional group represented by R in formulae (b-1) to (b-14) may be an electron donor site or an electron acceptor site, or the above-described polar group.

In the case where the dispersant is a compound having the electron donor site and the electron acceptor site, the polar group is preferably bonded to the electron acceptor site.

Further, because the dispersant may cause energy loss, it is preferably a compound having no triple bonds.

Examples of the dispersant include carboxyl group-containing indoline compounds, carboxyl group-containing oligothiophenes, and carboxyl group-containing coumarin compounds. Preferred among them are carboxyl group-containing indoline compounds and carboxyl group-containing oligothiophenes.

Specific examples of the dispersant include compounds having structures represented by the following formulae (1) to (8). In particular, a compound having a structure represented by formula (1) is preferred.

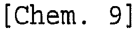

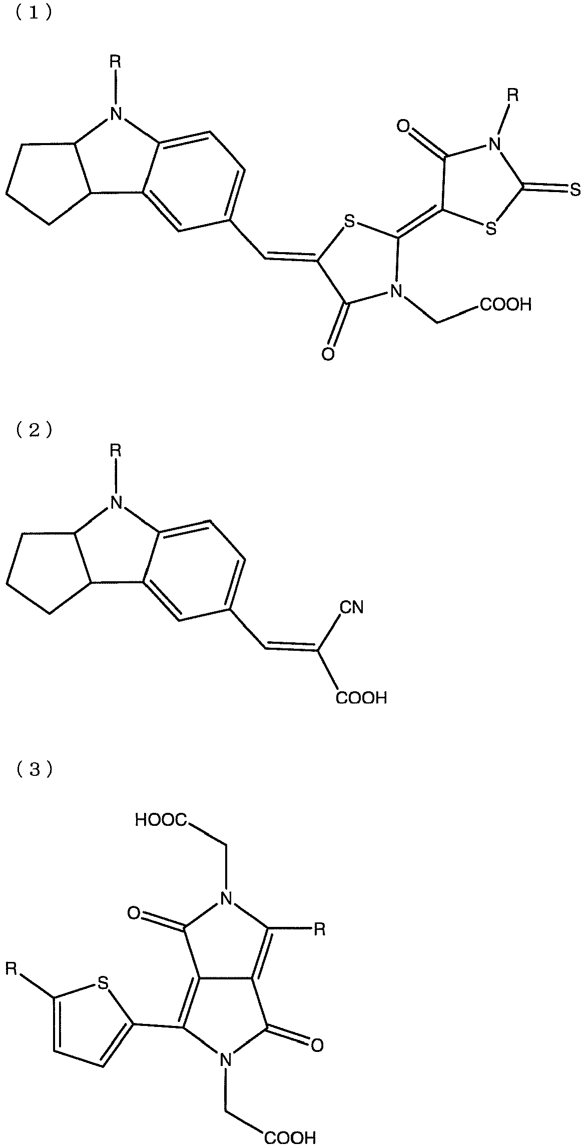

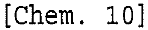

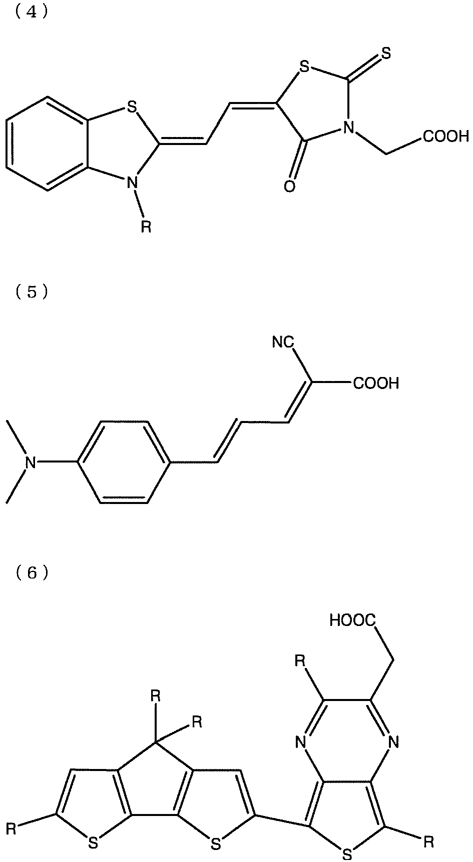

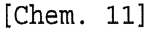

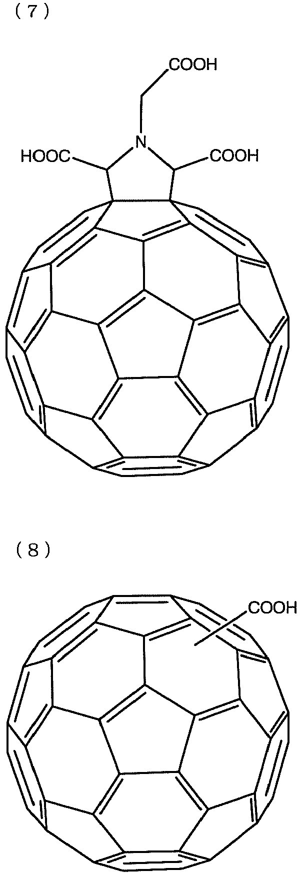

In formulae (1) to (8), R represents a hydrogen atom or a functional group. Examples of the functional group represented by R in formulae (1) to (8) include alkyl, aryl, alkoxy, alkenyl, alkynyl, and heteroaryl groups.

Examples of commercial products of the dispersant include D-149 and D-131 (both produced by Mitsubishi Paper Mills Ltd. , compounds having a structure with an aromatic ring and/or heterocyclic ring and one carboxyl group), NK-2684 and NK-2553 (both produced by Hayashibara Biochemical Laboratories, Inc., compounds having a structure with an aromatic ring and/or heterocyclic ring and one carboxyl group), carboxy group-containing methanofullerene (produced by Aldrich, a compound having a structure with an aromatic ring and/or heterocyclic ring and one carboxyl group), and C₆₀ Pyrrolidine tris-acid (produced by Aldrich, a compound having a structure with an aromatic ring and/or heterocyclic ring and three carboxyl groups).

The dispersant fulfills all of the following requirements (1) to (3) :
(1) the dispersant has a lower LUMO level than the organic semiconductor compound;
(2) solubility of the dispersant in the organic solvent is equal to or higher than solubility of the organic semiconductor compound in the organic solvent; and
(3) the dispersant has a higher HOMO level than the inorganic semiconductor compound.

(1) The dispersant has a lower LUMO level than the organic semiconductor compound.
   In the case where the dispersant has a higher LUMO level than the organic semiconductor compound, electrons present in the organic semiconductor compound are not transferred to the inorganic semiconductor compound in the active layer formed from the ink for an active layer of an organic solar cell, thus degrading the properties of the product as the solar cell. Although the LUMO level of the dispersant is not particularly limited and can be suitably selected according to the LUMO level of the inorganic semiconductor compound, the LUMO level is preferably -4.0 to -3.0 from the viewpoint of easily fulfilling the requirement (1).
   The term "LUMO level" as used herein refers to a value determined by measuring the HOMO level using an ionization potential measuring device, and subtracting from the HOMO level the band gap calculated from the ultraviolet-visible absorption spectrum.

(2) The solubility of the dispersant in the organic solvent is equal to or higher than solubility of the organic semiconductor compound in the organic solvent. The term "solubility in the organic solvent" as used herein refers to the amount of a solute that can be dissolved in 100 mL of an organic solvent at 23°C.
   In the case where the amount of the dispersant that can be dissolved in 100 mL of the organic solvent at 23°C is equal to or more than the amount of the organic semiconductor compound that can be dissolved in 100 mL of the organic solvent at 23°C, it can prevent a problem where all of the dispersant is deposited before the organic semiconductor compound is deposited during formation of an active layer using the ink for an active layer of an organic solar cell of the first aspect of the present invention. If all of the dispersant is deposited before the organic semiconductor compound is deposited, the effect of the dispersant will be lost, making it difficult to form an active layer in which the organic semiconductor compound and the inorganic semiconductor compound are extremely well dispersed.
   The solubility of the dispersant in the organic solvent is not particularly limited, and is selected according to the type of organic solvent used.

(3) The dispersant has a higher HOMO level than the inorganic semiconductor compound.
   In the case where the HOMO level of the dispersant is equal to or lower than that of the inorganic semiconductor compound, holes present in the dispersant are transferred to the inorganic semiconductor compound in the active layer formed from the ink for an active layer of an organic solar cell, causing the reverse hole transfer. As a result, the properties of the product as a solar cell are unfortunately degraded. Although the HOMO level of the dispersant is not particularly limited and may be suitably selected in accordance with the HOMO level of the organic semiconductor compound, the HOMO level is preferably -6.0 to -5.0 from the viewpoint of easily fulfilling the requirement (3).
   The term "HOMO level" as used herein refers to a value measured by an ionization potential measuring device.

The amount of the dispersant is not particularly limited. The preferred lower limit is 1 part by weight, and the preferred upper limit is 30 parts by weight, respectively, based on 100 parts by weight of the inorganic semiconductor compound. In the case where the amount of the dispersant is less than 1 part by weight, the effect obtained by adding the dispersant may be insufficient, and the active layer formed from the ink for an active layer of an organic solar cell may have low energy conversion efficiency. In the case where the amount of the dispersant is more than 30 parts by weight, the excess amount of the dispersant may impede the transfer of electrons or holes in the active layer formed from the ink for an active layer of an organic solar cell.
The more preferred lower limit of the amount of the dispersant is 2 parts by weight, and the more preferred upper limit of the amount of the dispersant is 20 parts by weight, based on 100 parts by weight of the inorganic semiconductor compound.

The combination of the organic semiconductor compound, the inorganic semiconductor compound, the organic solvent, and the dispersant is not particularly limited. In the case where the organic semiconductor compound is polyparaphenylene vinylene, it is preferred that the inorganic semiconductor compound be cadmium sulfide, the organic solvent be chlorobenzene, and the dispersant be NK-2684 (produced by Hayashibara Biochemical Laboratories, Inc., a compound having a structure with an aromatic ring and/or heterocyclic ring and one carboxyl group). In the case where the organic semiconductor compound is poly(3-hexylthiophene), it is preferred that the inorganic semiconductor compound be zinc oxide, the dispersant be D-149 (produced by Mitsubishi Paper Mills Ltd., a compound having a structure with an aromatic ring and/or heterocyclic ring and one carboxyl group), and the organic solvent be chloroform.

The method for producing the ink for an active layer of an organic solar cell of the first aspect of the present invention is not particularly limited. Examples include a method in which the organic semiconductor compound, the inorganic semiconductor compound, and the dispersant are dispersed and dissolved in the organic solvent using an ultrasonic disperser or the like to prepare an ink.

An active layer having high energy conversion efficiency can be stably and easily formed by the use of the ink for an active layer of an organic solar cell of the first aspect of the present invention.
An organic solar cell having an active layer produced using the ink for an active layer of an organic solar cell of the first aspect is also another aspect of the present invention.

In the active layer formed from the ink for an active layer of an organic solar cell of the first aspect of the present invention, the organic semiconductor compound and the inorganic semiconductor compound are extremely well dispersed. Thus, the area of the junction interface between the organic semiconductor compound and the inorganic semiconductor compound is large, and the active region for photocarrier generation is thus large. Accordingly, such an organic solar cell has high energy conversion efficiency. In the case where the inorganic semiconductor compound has an average particle size and a ratio of average particle size/average crystallite size in the above-described ranges, when electrons pass through the inorganic semiconductor compound in the active layer, the transfer of electrons is less likely to be impeded by grain boundaries, and electrons are smoothly collected at the electrode. Consequently, recombination of electrons and holes is inhibited, resulting in a further improvement in energy conversion efficiency.

The present invention also provides, as another aspect, a method for producing an organic solar cell using the ink for an active layer of an organic solar cell of the first aspect of the present invention, the method including the steps of applying the ink for an active layer of an organic solar cell of the first aspect of the present invention to a substrate having an electrode and drying the ink to form an active layer; and forming an electrode on the active layer.
The method for applying the ink for an active layer of an organic solar cell of the first aspect of the present invention is not particularly limited, and examples thereof include printing techniques such as spin coating. Because the organic semiconductor compound and the inorganic semiconductor compound have high dispersibility and a printing technique can be employed as the method for forming the active layer, the active layer can be stably and easily formed using the ink for an active layer of an organic solar cell of the first aspect of the present invention, and the cost of forming the active layer can be reduced.

Next, the organic solar cell of the second aspect of the present invention is described.
The organic solar cell of the second aspect of the present invention has an active layer in which an inorganic semiconductor compound is present in an organic semiconductor compound. The organic semiconductor compound and the inorganic semiconductor compound in the organic solar cell of the second aspect of the present invention may be the same as those used in the ink for an active layer of an organic solar cell of the first aspect of the present invention.

In the organic solar cell of the second aspect of the present invention, the inorganic semiconductor compound has an area ratio of 75 to 100% in a region from a cathode-side surface to a depth of 20% of a film thickness, in a cross-section of the active layer in a thickness direction.

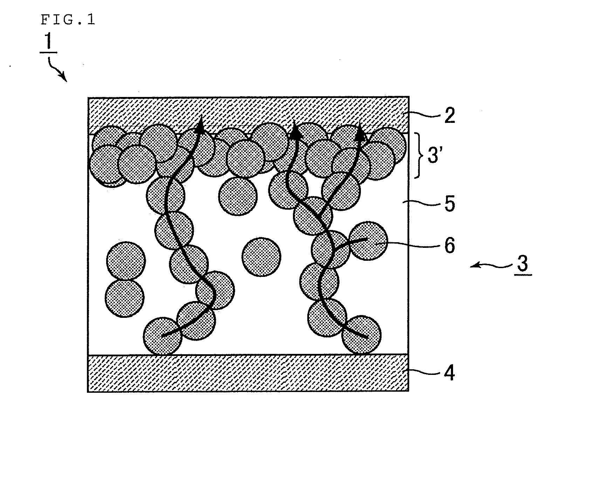

Fig. 1 shows an example of the organic solar cell of the second aspect of the present invention. In Fig. 1, an organic solar cell 1 includes a cathode 2, an active layer 3, and an anode 4. The active layer 3 has a structure such that an inorganic semiconductor compound 6 is present in an organic semiconductor compound 5.
In the organic solar cell shown in Fig. 1, the inorganic semiconductor compound 6 has an area ratio of 75 to 100% in a region 3' from the surface on the cathode 2 side to a depth of 20% of a film thickness of the active layer 3. Owing to the fact that the inorganic semiconductor compound 6 is present in a large amount near the cathode 2, electron paths (arrows) are easily formed, resulting in an increase in the photocurrent value and thus improving energy conversion efficiency. The area ratio is more preferably 80 to 100%, and still more preferably 90 to 100%.
The area ratio can be determined as follows, for example: an elemental mapping image of the cross section of the active layer 3 is prepared using FE-TEM (produced by Hitachi High-Technologies Corporation); subsequently, the region 3' from the surface on the cathode 2 side to a depth of 20% of a film thickness of the active layer 3 is determined; and the area ratio of the inorganic semiconductor compound 6 in the region 3' is calculated based on the mapped area.

In the organic solar cell of the second aspect of the present invention, the active layer is preferably such that the lower limit of the arithmetic average roughness of the cathode-side surface is 2.5 nm, and the upper limit of the arithmetic average roughness of the cathode-side surface is 20 nm, respectively. With the arithmetic average roughness in the above range, a better diffusion effect is achieved when incident light reflects at the interface with the cathode, making it possible to also effectively use the reflected light for photoelectric conversion.
With the arithmetic average roughness of less than 2.5 nm, the diffusion effect upon reflection of incident light may be difficult to achieve. With the arithmetic average roughness over 20 nm, sufficient adhesiveness may not be achieved when forming the cathode. The more preferred lower limit of the arithmetic average roughness is 10 nm, and the more preferred upper limit of the arithmetic average roughness is 18 nm.
The arithmetic average roughness can be measured by a method in accordance with JIS B 0601 (1994).

In the organic solar cell of the second aspect of the present invention, the preferred lower limit of the thickness of the active layer is 25 nm, and the preferred upper limit of the thickness of the active layer is 5 µm, respectively. In the case where the thickness of the active layer is less than 25 nm, a sufficient amount of photocarriers may not be generated. In the case where the thickness of the active layer is more than 5 µm, the distance until electrons generated at the anode are collected at the cathode may be long, and electrons and holes may thus be easily recombined.

Conventionally known products such as a glass substrate, an anode, a hole transport layer, and a cathode, other than the active layer, can be used as components of the organic solar cell of the second aspect of the present invention.

The organic solar cell of the second aspect of the present invention can be produced, for example, by a method including a step of applying a cathode-side active layer ink containing the inorganic semiconductor compound in an amount of 75 to 100 vol% based on the volume of the organic semiconductor compound such that the ink has a thickness extending from a cathode-side surface to a depth of 50% or less of a film thickness of the active layer, and drying the ink to form a cathode-side active layer. The film thickness of the cathode-side active layer is preferably 40% or less, more preferably 30% or less, still more preferably 20% or less, and particularly preferably 10% or less, of the thickness of the active layer. Such a method for producing the organic solar cell is also another aspect of the present invention.

The method for producing the organic solar cell of the second aspect of the present invention may further include, before or after the step of forming the cathode-side active layer, a step of applying an anode-side active layer ink containing the inorganic semiconductor compound in an amount of 25 to 75 vol% based on the volume of organic semiconductor compound and drying the ink to form an anode-side active layer.
In such a method for producing the organic solar cell, the use of the anode-side active layer ink and the cathode-side active layer ink containing the inorganic semiconductor compound in addition to the organic semiconductor compound allows these inks to be applied in an overlapping manner. In other words, even after a coating film is formed from one of these inks, it is possible to apply the other ink to the coating film. This allows a large amount of inorganic semiconductor compound to be present near the cathode.
In contrast, for example, in the case of producing an organic solar cell having a p-type organic semiconductor and an n-type organic semiconductor, because each ink for an active layer does not contain an inorganic semiconductor compound, an attempt to apply one of the inks to a coating film formed from the other ink causes the lower coating film to be dissolved by an organic solvent, making it difficult for these inks to be successfully applied in an overlapping manner.

The anode-side active layer ink and the cathode-side active layer ink may also contain, in addition to the organic semiconductor compound and the inorganic semiconductor compound, components such as an organic solvent and a dispersant used in the ink for an active layer of an organic solar cell of the first aspect of the present invention.
The method for applying the anode-side active layer ink and the method for applying the cathode-side active layer ink are not particularly limited. Examples include printing techniques such as spin coating.

The method for producing the organic solar cell of the second aspect of the present invention may include a step in which a solvent that dissolves the organic semiconductor compound is applied to the cathode-side surface of the active layer to partially remove the organic semiconductor compound, and the solvent is then dried, thereby exposing the inorganic semiconductor compound.
It is possible to adjust the arithmetic average roughness of the cathode-side surface of the active layer by such a step. As a result, a better diffusion effect is achieved when incident light reflects at the interface with the cathode, thus making it possible to also effectively use the reflected light for photoelectric conversion.

Examples of the solvent that dissolves the organic semiconductor compound include chloroform, chlorobenzene, ortho-dichlorobenzene, toluene, and xylene.
Examples of the method for applying the solvent that dissolves the organic semiconductor compound include a method that uses spin coating.

### - Advantageous Effects of the Invention

The present invention provides an ink for an active layer of an organic solar cell, wherein an active layer having high energy conversion efficiency can be easily and stably formed with the ink; an organic solar cell having high energy conversion efficiency; and a method for producing the organic solar cell.

### BRIEF DESCRIPTION OF DRAWINGS

Fig. 1 is a cross-sectional view showing an example of an organic solar cell of the second aspect of the present invention.

### DESCRIPTION OF EMBODIMENTS

The first aspect of the present invention is described in further detail with reference to examples below; however, the present invention is not limited to these examples.

### (Example 1)

### (Preparation of Particles of the Inorganic Semiconductor Compound)

One part by weight of zinc acetate dihydrate was dissolved in 35 parts by weight of methanol. While stirring the mixture in a hot-water bath at 60°C, a solution of 0.5 parts by weight of potassium hydroxide in 15 parts by weight of methanol was added dropwise to the mixture. After dropwise addition, the mixture was continuously stirred for 5 hours to obtain ZnO nanoparticle dispersion. Next, the ZnO nanoparticle dispersion was centrifuged, the supernatant was removed, and the precipitate was collected. ZnO nanoparticles were thus obtained.
The obtained ZnO nanoparticles were dispersed in methanol, and the average particle size of the resultant dispersion was measured using a dynamic light scattering analyzer (380DLS produced by PSS-NICOMP). The resultant ZnO nanoparticles were also measured using an X-ray diffractometer (RINT1000 produced by Rigaku Corporation) to obtain a peak, and a half width from which a device-dependent value was subtracted was determined from the peak. Then, the average crystallite size was determined using Scherrer's equation shown below. The results are shown in a table.
D = Kλ/βcosθ
D: Crystallite diameter
λ: Wavelength of the measured X-ray
β (rad) : broadening of the diffraction line due to the crystallite size (half width)
θ (rad) : Angle of the measured peak
K: Scherrer constant

### (Production of the Ink for an Active Layer of an Organic Solar Cell)

Eight parts by weight of poly(3-alkylthiophene) (LUMO level of -3.0, and 2% by weight solubility in chloroform), 24 parts by weight of ZnO nanoparticles (HOMO level of -7.5), and 2 parts by weight of D-149 as a dispersant (produced by Mitsubishi Paper Mills Ltd., a compound having a structure with an aromatic ring and/or heterocyclic ring and one carboxyl group, LUMO level of -3.2, HOMO level of -5.2, and 2% by weight solubility in chloroform) were dispersed and dissolved in 1,000 parts by weight of chloroform to prepare an ink for an active layer of an organic solar cell.
The dispersant used was described in a table in regard to the presence of a nitrogen atom, a sulfur atom, a fluorine atom, or a carbonyl group at a position other than the position of a polar group bonded to the structure; the presence of an electron donor site and an electron acceptor site; and the presence of a triple bond.

### (Production of the Organic Solar Cell)

An ITO film having a thickness of 240 nm was formed as the anode on a glass substrate, ultrasonically cleaned in acetone, methanol, and isopropyl alcohol in the stated order for 10 minutes each, and dried. A layer of polyethylene dioxythiophene:polystyrene sulfonate (PEDOT:PSS) having a thickness of 100 nm was formed as a hole transport layer on the surface of the ITO film by spin coating. Next, the above-prepared ink for an active layer of an organic solar cell was applied to the surface of the hole transport layer to a thickness of 100 nm by spin coating to form an active layer. Further, an aluminum film having a thickness of 100 nm was formed as the cathode on the surface of the active layer by vacuum deposition. An organic solar cell was thus prepared.
The appearance of the aluminum electrode of the produced organic solar cell was observed, and the dispersibility of the organic semiconductor compound and the inorganic semiconductor compound was evaluated according to three ratings (○, Δ, ×). When the aluminum electrode of the prepared organic solar cell has a mirror-like appearance, it means that the organic semiconductor compound and the inorganic semiconductor compound in the active layer formed below the electrode are dispersed at the nano level. In this case, the dispersibility was indicated by "o" in the table. When the aluminum electrode is white, it means that the dispersibility is insufficient at the nano level. In this case, the dispersibility was indicated by "×" in the table.

### (Example 2)

Eight parts by weight of poly(3-alkylthiophene) (LUMO level of -3.0, and 2% by weight of solubility in chlorobenzene), 24 parts by weight of the ZnO nanoparticles obtained in Example 1 (HOMO level of -7.5), and 1 part by weight of NK-2684 (produced by Hayashibara Biochemical Laboratories, Inc., a compound having a structure with an aromatic ring and/or heterocyclic ring and one carboxyl group, LUMO level of -3.2, HOMO level of -5.6, and 2% by weight solubility in chlorobenzene) as a dispersant were dispersed and dissolved in 800 parts by weight of chlorobenzene to prepare an ink for an active layer of an organic solar cell.
An organic solar cell was prepared in the same manner as in Example 1 except that the above-prepared ink for an active layer of an organic solar cell was used.

### (Example 3)

An ink for an active layer of an organic solar cell was prepared in the same manner as in Example 1 except that 1 part by weight of NK-2553 (produced by Hayashibara Biochemical Laboratories, Inc., a compound having a structure with an aromatic ring and/or heterocyclic ring and one carboxyl group, LUMO level of -3.2, HOMO level of -5.6, and 2% by weight solubility in chlorobenzene) was used as a dispersant and chlorobenzene was used as an organic solvent.
An organic solar cell was prepared in the same manner as in Example 1 except that the above-prepared ink for an active layer of an organic solar cell was used.

### (Example 4)

An ink for an active layer of an organic solar cell was prepared in the same manner as in Example 1, except that 1 part by weight of D-131 (produced by Mitsubishi Paper Mills Ltd., a compound having a structure with an aromatic ring and/or heterocyclic ring and one carboxyl group, LUMO level of -3.3, HOMO level of -5.6, and 2% by weight solubility in chlorobenzene) was used as a dispersant and chlorobenzene was used as an organic solvent.
An organic solar cell was prepared in the same manner as in Example 1 except that the above-prepared ink for an active layer of an organic solar cell was used.

### (Example 5)

An ink for an active layer of an organic solar cell was prepared in the same manner as in Example 1, except that 1 part by weight of carboxy group-containing methanofullerene (produced by Aldrich, a compound having a structure with an aromatic ring and/or heterocyclic ring and one carboxyl group, LUMO level of -3.9, HOMO level of -6.0, and 2% by weight solubility in a chloroform-pyridine mixed solvent (9:1 in weight ratio)) was used as a dispersant and a chloroform-pyridine mixed solvent (9:1 in weight ratio) was used as an organic solvent.
An organic solar cell was prepared in the same manner as in Example 1 except that the above-prepared ink for an active layer of an organic solar cell was used.

### (Example 6)

An ink for an active layer of an organic solar cell was prepared in the same manner as in Example 1 except that 1 part by weight of C₆₀ Pyrrolidine tris-acid(produced by Aldrich, a compound having a structure with an aromatic ring and/or heterocyclic ring and 3 carboxyl groups, LUMO level of -3.9, HOMO level of -6.0, and 2% by weight solubility in a chloroform-pyridine mixed solvent (9:1 in weight ratio)) was used as a dispersant and a chloroform-pyridine mixed solvent (9:1 in weight ratio) was used as an organic solvent.
An organic solar cell was prepared in the same manner as in Example 1 except that the above-prepared ink for an active layer of an organic solar cell was used.

### (Example 7)

ZnO nanoparticles were prepared in the same manner as in Example 1, except that the reaction was carried out in a hot-water bath at 35°C for 72 hours during the preparation of ZnO nanoparticles.
An organic solar cell was prepared in the same manner as in Example 1 except that the above-prepared ZnO nanoparticles were used.

### (Example 8)

ZnO nanoparticles were prepared in the same manner as in Example 1, except that the reaction was carried out at room temperature (25°C) for 96 hours, without using a hot-water bath during the preparation of ZnO nanoparticles.
An organic solar cell was prepared in the same manner as in Example 1 except that the above-prepared ZnO nanoparticles were used.

### (Comparative Example 1)

An organic solar cell was prepared in the same manner as in Example 1, except that a dispersant was not used.

### (Comparative Example 2)

An ink for an active layer of an organic solar cell was prepared in the same manner as in Example 1, except that 2 parts by weight of HKX-2587 (produced by Hayashibara Biochemical Laboratories, Inc., a compound having a structure with an aromatic ring and/or heterocyclic ring and a carboxyl group asymmetrically bonded to the structure, LUMO level of -3.1, HOMO level of -5.3, and 0.5% by weight solubility in chloroform) was used as a dispersant.
An organic solar cell was prepared in the same manner as in Example 1 except that the above-prepared ink for an active layer of an organic solar cell was used.

### (Comparative Example 3)

An ink for an active layer of an organic solar cell was prepared in the same manner as in Example 1, except that 2 parts by weight of MK-2 (produced by Soken Chemical & Engineering Co., Ltd., a compound having a structure with an aromatic ring and/or heterocyclic ring and a carboxyl group asymmetrically bonded to the structure, LUMO level of -2.8, HOMO level of -5.1, and 3% by weight solubility in chloroform) was used as a dispersant.
An organic solar cell was prepared in the same manner as in Example 1 except that the above-prepared ink for an active layer of an organic solar cell was used.

### (Comparative Example 4)

An ink for an active layer of an organic solar cell was prepared in the same manner as in Example 1, except that 2 parts by weight of a polyalkoxythiophene derivative (produced by Aldrich, a compound having a polythiophene structure and an alkoxy group symmetrically bonded to the structure, LUMO level of -3.1, HOMO level of -5.0, and 2% by weight solubility in chloroform) was used as a dispersant.
An organic solar cell was prepared in the same manner as in Example 1 except that the above-prepared ink for an active layer of an organic solar cell was used.

### (Comparative Example 5)

An ink for an active layer of an organic solar cell was prepared in the same manner as in Example 1, except that 2 parts by weight of a silicon phthalocyanine compound (produced by Aldrich, a compound having no polar groups, LUMO level of -3.5, HOMO level of -5.0, and 2% by weight solubility in chloroform) was used as a dispersant.
An organic solar cell was prepared in the same manner as in Example 1 except that the above-prepared ink for an active layer of an organic solar cell was used.

### (Comparative Example 6)

An ink for an active layer of an organic solar cell was prepared in the same manner as in Example 1, except that 2 parts by weight of tetracarboxy copper phthalocyanine (produced by Aldrich, a compound having a phthalocyanine structure and a carboxyl group symmetrically bonded to the structure, LUMO level of -3.3, HOMO level of -4.8, and 2% by weight solubility in a chloroform-pyridine mixed solvent (9:1 in weight ratio)) was used as a dispersant, and 800 parts by weight of a chloroform-pyridine mixed solvent (9:1 in weight ratio) was used as an organic solvent.
An organic solar cell was prepared in the same manner as in Example 1 except that the above-prepared ink for an active layer of an organic solar cell was used.

### (Comparative Example 7)

An ink for an active layer of an organic solar cell was prepared in the same manner as in Example 1, except that 2 parts by weight of N-719 (a ruthenium dye produced by Aldrich, a compound having a carboxyl group symmetrically bonded to dye structure, LUMO level of -4.0, HOMO level of -5.6, and 2% by weight solubility in a chloroform-pyridine mixed solvent (9:1 in weight ratio)) was used as a dispersant, and 800 parts by weight of a chloroform-pyridine mixed solvent (9:1 in weight ratio) was used.
An organic solar cell was prepared in the same manner as in Example 1 except that the above-prepared ink for an active layer of an organic solar cell was used.

### <Evaluation 1>

### (Measurement of the Area Ratio of the Inorganic Semiconductor Compound)

The cross section of the prepared organic solar cell was observed using FE-TEM (produced by Hitachi High-Technologies Corporation), and an elemental mapping image of zinc was thus obtained. Using the obtained elemental mapping image, the area ratio of the inorganic semiconductor compound in a region from the cathode-side surface to a depth of 20% of a film thickness was calculated. The area ratio of zinc oxide can be determined by measuring the area ratio of zinc. The results are shown in a table.

### <Evaluation 2>

### (Measurement of Energy Conversion Efficiency)

A voltage source (model 236 produced by Keithley) was connected between the electrodes of each organic solar cell obtained in Examples and Comparative Examples, and the energy conversion efficiency of each organic solar cell was measured using a solar simulator (produced by Yamashita Denso Corporation) with an intensity of 100 mW/cm². The energy conversion efficiency of the organic solar cell prepared in Comparative Example 1 was standardized as 1.00. The results are shown in a table.

**Table 1**

| | Organic semiconductor compound | | Inorganic semiconductor compound | | | | Dispersant | | | | | | | | | |
|---|---|---|---|---|---|---|---|---|---|---|---|---|---|---|---|---|
| | LUMO level | Solubility (wt%) | (A) Average particle diameter (nm) | (B) Average crystallite diameter (nm) | A/B | HOMO level | Aromatic ring and/or heterocyclic ring | Polar group | Symmetry | N, S, or F atom | Carbonyl group | Electron acceptor and electron donor sites | Triple bond | LUMO level | Solubility (wt%) | HOMO level |
| Example 1 | -3.0 | 2 | 9.7 | 5.8 | 1.67 | -7.5 | Present | Carboxyl group | Asymmetry | Present | Present | Present | Absent | -3.2 | 2 | -5.2 |
| Example 2 | -3.0 | 2 | 9.7 | 5.8 | 1.67 | -7.5 | Present | Carboxyl group | Asymmetry | Present | Present | Present | Absent | -3.2 | 2 | -5.6 |
| Example 3 | -3.0 | 2 | 9.7 | 5.8 | 1.67 | -7.5 | Present | Carboxyl group | Asymmetry | Present | Absent | Present | Present | -3.2 | 2 | -5.6 |
| Example 4 | -3.0 | 2 | 9.7 | 5.8 | 1.67 | -7.5 | Present | Carboxyl group | Asymmetry | Present | Absent | Present | Present | -3.3 | 2 | -5.6 |
| Example 5 | -3.0 | 2 | 9.7 | 5.8 | 1.67 | -7.5 | Present | Carboxyl group | Asymmetry | Absent | Absent | Absent | Absent | -3.9 | 2 | -6.0 |
| Example 6 | -3.0 | 2 | 9.7 | 5.8 | 1.67 | -7.5 | Present | Carboxyl group | Asymmetry | Present | Absent | Absent | Absent | -3.9 | 2 | -6.0 |
| Example 7 | -3.0 | 2 | 5.8 | 1.7 | 3.41 | -7.5 | Present | Carboxyl group | Asymmetry | Present | Present | Present | Absent | -3.2 | 2 | -5.2 |
| Example 8 | -3.0 | 2 | 4.3 | 0.9 | 4.78 | -7.5 | Present | Carboxyl group | Asymmetry | Present | Present | Present | Absent | -3.2 | 2 | -5.2 |
| Comparative Example 1 | -3.0 | 2 | 9.7 | 5.8 | 1.67 | -7.5 | - | - | - | - | - | - | - | - | - | - |
| Comparative Example 2 | -3.0 | 2 | 9.7 | 5.8 | 1.67 | -7.5 | Present | Carboxyl group | Asymmetry | Present | Absent | Present | Present | -3.1 | 0.5 | -5.3 |
| Comparative Example 3 | -3.0 | 2 | 9.7 | 5.8 | 1.67 | -7.5 | Present | Carboxyl group | Asymmetry | Present | Absent | Present | Present | -2.8 | 3 | -5.1 |
| Comparative Example 4 | -3.0 | 2 | 9.7 | 5.8 | 1.67 | -7.5 | Present | Alkoxy group | Symmetry | Present | Absent | Absent | Absent | -3.1 | 2 | -5.0 |
| Comparative Example 5 | -3.0 | 2 | 9.7 | 5.8 | 1.67 | -7.5 | Present | Absent | - | Present | Absent | Absent | Absent | -3.5 | 2 | -5.0 |
| Comparative Example 6 | -3.0 | 2 | 9.7 | 5.8 | 1.67 | -7.5 | Present | Carboxyl group | Symmetry | Present | Absent | Absent | Absent | -3.3 | 2 | -4.8 |
| Comparative Example 7 | -3.0 | 2 | 9.7 | 5.8 | 1.67 | -7.5 | Present | Carboxyl group | Symmetry | Present | Absent | Absent | Absent | -4.0 | 2 | -5.6 |

**Table 2**

| | Dispersibility | Area ratio (%) of the inorganic semiconductor compound in the mapping image | Energy conversion efficiency |
|---|---|---|---|
| Example 1 | ○ | 69.6 | 1.22 |
| Example 2 | ○ | 68.3 | 1.15 |
| Example 3 | ○ | 69.2 | 1.10 |
| Example 4 | ○ | 70.3 | 1.10 |
| Example 5 | ○ | 66.7 | 1.05 |
| Example 6 | ○ | 68.2 | 1.07 |
| Example 7 | ○ | 71.2 | 0.94 |
| Example 8 | ○ | 69.2 | 0.87 |
| Comparative Example 1 | × | 65.1 | 1.00 |
| Comparative Example 2 | × | 65.2 | 0.87 |
| Comparative Example 3 | ○ | 70.2 | 0.56 |
| Comparative Example 4 | × | 66.8 | 0.67 |
| Comparative Example 5 | × | 64.2 | 0.44 |
| Comparative Example 6 | Δ | 67.3 | 0.54 |
| Comparative Example 7 | Δ | 68.5 | 0.88 |

The second aspect of the present invention is described in further detail with reference to the examples below; however, the present invention is not limited to these examples.

### (Example 9)

### (Preparation of Particles of the Inorganic Semiconductor Compound)

ZnO nanoparticles were prepared in the same manner as in Example 1.

### (Production of the Organic Solar Cell)

An ITO film as the anode and a hole transport layer were formed on a glass substrate in the same manner as in Example 1.
Next, 5 parts by weight of the prepared ZnO nanoparticles, 2 parts by weight of poly(3-alkylthiophene), and 0.5 parts by weight of D-149 (produced by Mitsubishi Paper Mills Ltd., a compound having a structure with an aromatic ring and/or heterocyclic ring and one carboxyl group, LUMO level of -3.2, HOMO level of -5.2, and 2% by weight solubility in chloroform) as a dispersant were dissolved and dispersed in 343 parts by weight of chloroform to prepare an anode-side active layer ink. The prepared anode-side active layer ink was applied to the hole transport layer to a thickness of 80 nm by spin coating and dried to form an anode-side active layer.
Further, 28.5 parts by weight of the prepared ZnO nanoparticles and 1 part by weight of poly(3-alkylthiophene) were dissolved and dispersed in a mixed solvent of 1373.2 parts by weight of chloroform and 72.3 parts by weight of methanol to prepare a cathode-side active layer ink. The prepared cathode-side active layer ink was applied to the anode-side active layer to a thickness of 20 nm by spin coating and dried, thus forming an active layer including the anode-side active layer and the cathode-side active layer.
Further, aluminum was applied as the cathode to the surface of the active layer to a thickness of 100 nm by vacuum deposition, and an organic solar cell was thus produced.

### (Example 10)

An organic solar cell was produced in the same manner as in Example 9 except that only 1.00 part by weight of the prepared ZnO nanoparticles was dissolved and dispersed in a mixed solvent of 46.6 parts by weight of chloroform and 2.45 parts by weight of methanol to prepare a cathode-side active layer ink.

### (Example 11)

An organic solar cell was produced in the same manner as in Example 9 except that 15.0 parts by weight of the prepared ZnO nanoparticles and 1.00 part by weight of poly (3-alkylthiophene) were dissolved and dispersed in a mixed solvent of 744.8 parts by weight of chloroform and 39.2 parts by weight of methanol to prepare a cathode-side active layer ink.

### (Example 12)

An organic solar cell was produced in the same manner as in Example 9 except that a dispersant was not added to the anode-side active layer ink.

### (Example 13)

An organic solar cell was produced in the same manner as in Example 12 except that only 1.00 part by weight of the prepared ZnO nanoparticles was dissolved and dispersed in a mixed solvent of 46.6 parts by weight of chloroform and 2.45 parts by weight of methanol to prepare a cathode-side active layer ink.

### (Example 14)

### (Preparation of Particles of the Inorganic Semiconductor Compound)

ZnO nanoparticles were prepared in the same manner as in Example 1.

### (Production of the Organic Solar Cell)

An ITO film having a thickness of 240 nm was formed as the cathode on a glass substrate, ultrasonically cleaned in acetone, methanol, and isopropyl alcohol in the stated order for 10 minutes each, and dried. A titanium oxide thin film having a thickness of 10 nm was formed as an electron transport layer on the surface of the ITO film by spin coating an ethanol solution of titanium isopropoxide.
Next, 28.5 parts by weight of the prepared ZnO nanoparticles and 1 part by weight of poly(3-alkylthiophene) were dissolved and dispersed in a mixed solvent of 1373.2 parts by weight of chloroform and 72.3 parts by weight of methanol to prepare a cathode-side active layer ink. The prepared cathode-side active layer ink was applied to the surface of the electron transport layer to a thickness of 20 nm by spin coating and dried to form a cathode-side active layer.
Further, 5 parts by weight of ZnO nanoparticles, 2 parts by weight of poly(3-alkylthiophene), and 0.5 parts by weight of D-149 (produced by Mitsubishi Paper Mills Ltd., a compound having a structure with an aromatic ring and/or heterocyclic ring and one carboxyl group, LUMO level of -3.2, HOMO level of -5.2, and 2% by weight solubility in chloroform) as a dispersant were dissolved and dispersed in 343 parts by weight of chloroform to prepare an anode-side active layer ink. The prepared anode-side active layer ink was applied to the cathode-side active layer to a thickness of 80 nm by spin coating and dried, thus forming an active layer including the cathode-side active layer and the anode-side active layer.
Further, a layer of molybdenum oxide having a thickness of 10 nm was formed as the anode on the surface of the active layer by vacuum deposition, and subsequently, silver was applied to a thickness of 100 nm. An organic solar cell was thus produced.

### (Comparative Example 8)

An organic solar cell was prepared in the same manner as in Example 12, except that 15.0 parts by weight of the prepared ZnO nanoparticles and 1.00 part by weight of poly(3-alkylthiophene) were dissolved and dispersed in a mixed solvent of 744.8 parts by weight of chloroform and 39.2 parts by weight of methanol to prepare a cathode-side active layer ink.

### (Comparative Example 9)

An ITO film having a thickness of 240 nm was formed as the cathode on a glass substrate, ultrasonically cleaned in acetone, methanol, and isopropyl alcohol in the stated order for 10 minutes each, and dried. A titanium oxide thin film having a thickness of 10 nm was formed as an electron transport layer on the surface of the ITO film by spin coating an ethanol solution of titanium isopropoxide.
Next, the ink for an active layer of an organic solar cell prepared in Example 1 was applied to the surface of the electron transport layer to a thickness of 100 nm by spin coating and dried to prepare an active layer. Further, a layer of molybdenum oxide having a thickness of 10 nm was formed as the anode on the surface of the active layer by vacuum deposition, and subsequently, silver was applied to a thickness of 100 nm. An organic solar cell was thus produced.

The prepared organic solar cell was evaluated in the same manner as in <Evaluation 1> and <Evaluation 2> described above.

**Table 3**

| | Organic semiconductor compound | | Inorganic semiconductor compound | | | | Dispersant | | | | | | | | | |
|---|---|---|---|---|---|---|---|---|---|---|---|---|---|---|---|---|
| | LUMO level | Solubility (wt%) | (A) Average particle diameter (nm) | (B) Average crystallite diameter (nm) | A/B | HOMO level | Aromatic ring and/or heterocyclic ring | Polar group | Symmetry | N, S, or F atom | Carbonyl group | Electron acceptor and electron donor sites | Triple bond | (LUMO level | Solubility (wt%) | HOMO level |
| Example 9 | -3.0 | 2 | 9.7 | 5.8 | 1.67 | -7.5 | Present | Carboxyl group | Asymmetry | Present | Present | Present | Absent | -3.2 | 2 | -5.2 |
| Example 10 | -3.0 | 2 | 9.7 | 5.8 | 1.67 | -7.5 | Present | Carboxyl group | Asymmetry | Present | Present | Present | Absent | -3.2 | 2 | -5.2 |
| Example 11 | -3.0 | 2 | 9.7 | 5.8 | 1.67 | -7.5 | Present | Carboxyl group | Asymmetry | Present | Present | Present | Absent | -3.2 | 2 | -5.2 |
| Example 12 | -3.0 | 2 | 9.7 | 5.8 | 1.67 | -7.5 | - | - | - | - | - | - | - | - | - | - |
| Example 13 | -3.0 | 2 | 9.7 | 5.8 | 1.67 | -7.5 | - | - | - | - | - | - | - | - | - | - |
| Example 14 | -3.0 | 2 | 9.7 | 5.8 | 1.67 | -7.5 | Present | Carboxyl group | Asymmetry | Present | Present | Present | Absent | -3.2 | 2 | -5.2 |
| Comparative Example 8 | -3.0 | 2 | 9.7 | 5.8 | 1.67 | -7.5 | - | - | - | - | - | - | - | - | - | - |
| Comparative Example 9 | -3.0 | 2 | 9.7 | 5.8 | 1.67 | -7.5 | Present | Carboxyl group | Asymmetry | Present | Present | Present | Absent | -3.2 | 2 | -5.2 |

**Table 4**

| | Dispersibility | Area ratio (%) of the inorganic semiconductor compound in the mapping image | Energy conversion efficiency |
|---|---|---|---|
| Example 9 | ○ | 83.9 | 1.45 |
| Example 10 | ○ | 98.2 | 1.51 |
| Example 11 | ○ | 76.7 | 1.31 |
| Example 12 | × | 81.2 | 1.19 |
| Example 13 | × | 99.8 | 1.24 |
| Example 14 | ○ | 82.3 | 1.28 |
| Comparative Example 8 | ○ | 74.2 | 1.07 |
| Comparative Example 9 | × | 69.6 | 1.09 |

### INDUSTRIAL APPLICABILITY

The present invention provides an ink for an active layer of an organic solar cell, wherein an active layer having high energy conversion efficiency can be stably and easily formed from the ink; an organic solar cell having high energy conversion efficiency; and a method for producing the organic solar cell.

### REFERENCE SIGNS LIST

- 1: Organic solar cell
- 2: Cathode
- 3: Active layer
- 3': Region from a cathode-side surface of an active layer to a depth of 20% of a film thickness
- 4: Anode
- 5: Organic semiconductor compound
- 6: Inorganic semiconductor compound

## Claims

1. An ink for an active layer of an organic solar cell, the ink comprising:
an organic semiconductor compound;
an inorganic semiconductor compound;
an organic solvent; and
a dispersant;
said dispersant being a compound having a structure with an aromatic ring and/or heterocyclic ring and a polar group asymmetrically bonded to the structure, and fulfilling all of the following requirements (1) to (3):
(1) said dispersant has a lower LUMO level than said organic semiconductor compound;
(2) solubility of said dispersant in said organic solvent is equal to or higher than solubility of said organic semiconductor compound in said organic solvent; and
(3) said dispersant has a higher HOMO level than said inorganic semiconductor compound.

2. The ink for an active layer of an organic solar cell according to claim 1, wherein the dispersant is a compound having a nitrogen atom, a sulfur atom, a fluorine atom, or a carbonyl group at a position other than the position of the polar group bonded to the structure.

3. The ink for an active layer of an organic solar cell according to claim 2, wherein the dispersant is a compound having a carbonyl group at a position other than the position of the polar group bonded to the structure.

4. The ink for an active layer of an organic solar cell according to claim 1, 2, or 3, wherein the dispersant has an electron donor site and an electron acceptor site, and the polar group is bonded to said electron acceptor site.

5. The ink for an active layer of an organic solar cell according to claim 1, 2, 3, or 4, wherein the dispersant is a compound without a triple bond.

6. The ink for an active layer of an organic solar cell according to claim 1, 2, 3, 4, or 5, wherein the polar group in the dispersant is a carboxyl group.

7. The ink for an active layer of an organic solar cell according to claim 1, 2, 3, 4, 5, or 6, wherein the dispersant is a compound having a structure represented by following formula (1):

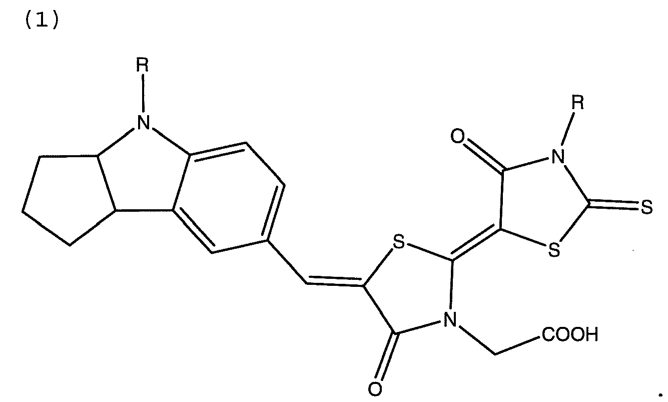

8. The ink for an active layer of an organic solar cell according to claim 1, 2, 3, 4, 5, 6, or 7, wherein the inorganic semiconductor compound has an average particle size of 1 to 50 nm and a ratio of average particle size/average crystallite size of 1 to 3.

9. An organic solar cell comprising an active layer produced using the ink for an active layer of an organic solar cell according to claim 1, 2, 3, 4, 5, 6, 7, or 8.

10. A method for producing an organic solar cell using the ink for an active layer of an organic solar cell according to claim 1, 2, 3, 4, 5, 6, 7, or 8, the method comprising the steps of:
applying said ink for an active layer of an organic solar cell to a substrate having an electrode and drying the ink to form an active layer; and
forming an electrode on said active layer.

11. An organic solar cell having an active layer in which an inorganic semiconductor compound is present in an organic semiconductor compound,
wherein in a cross-section of said active layer in a thickness direction, said inorganic semiconductor compound has an area ratio of 75 to 100% in a region from a cathode-side surface to a depth of 20% of a film thickness.

12. A method for producing the organic solar cell according to claim 11, the method comprising the step of:
applying a cathode-side active layer ink containing the inorganic semiconductor compound in an amount of 75 to 100 vol% based on the volume of the organic semiconductor compound such that the ink has a thickness extending from a cathode-side surface to a depth of 50% or less of a film thickness of the active layer, and drying the ink to form a cathode-side active layer.
